# NEW EUROPEAN PATENT SPECIFICATION

(11) **EP 2 574 327 B2**
(45) Date of publication and mention of the opposition decision: **07.03.2018**
(45) Mention of the grant of the patent: 18.03.2015
(21) Application number: 11194272.8
(22) Date of filing: 19.12.2011
(51) Int. Cl.: A61K 8/02, A61K 8/04, A61K 8/20, A61K 8/26, A61Q 15/00

(54) **Antiperspirant compositions and method for reducing perspiration**
Schweißhemmende Zusammensetzungen und Verfahren zur Reduktion der Schweißbildung
Compositions anti-transpirantes et procédé pour réduire la transpiration

(30) Priority: 28.09.2011 EP 11183152
(43) Date of publication of application: 03.04.2013
(60) Divisional application: 14160729.1
(73) Proprietor: Unilever PLC, London EC4Y 0DY (GB); Unilever N.V., 3013 AL Rotterdam (NL)
(72) Inventor: Baker, Michael Richard, Wirral, Merseyside CH63 3JW (GB); Fletcher, Neil Robert, Wirral, Merseyside CH63 3JW (GB); Shafran, Kirill, Wirral, Merseyside CH63 3JW (GB); Franklin, Kevin Ronald, Wirral, Merseyside CH63 3JW (GB)
(74) Representative: Whaley, Christopher

(56) References cited:
- EP-A1- 1 974 716
- EP-A1- 1 974 716
- WO-A2-02/069914
- WO-A2-2011/076569
- US-A- 5 955 065
- US-A1- 2006 099 161
- US-A1- 2010 047 296
- C. D. VAUGHAN: "Using solubility parameters in cosmetics formulation", J. SOC. COSMET. CHEM., vol. 36, September 1985 (1985-09), pages 319-333, XP809880,
- VAUGHAN C D: "SOLUBILITY EFFECTS IN PRODUCT, PACKAGE, PENETRATION AND PRESERVATION", COSMETICS & TOILETRIES, WHEATON, IL, US, vol. 103, 1 October 1988 (1988-10-01), XP000872394, ISSN: 0361-4387
- "Cosmetics and Drugs- Medicinal Mineral Salts ED - Bennett H", 1 January 2004 (2004-01-01), TWO THOUSAND FORMULAS, RECIPES & TRADE SECRETS: THE CLASSIC DO-IT-YOURSELF BOOK OF PRACTICAL EVERYDAY CHEMISTRY, FERAL HOUSE, PAGE(S) 73, XP009171485, ISBN: 0-922915-95-4

## Description

The present Invention is in the field of cosmetic compositions, in particular antiperspirant compositions and their use in reducing perspiration.

A variety of antiperspirant compositions have been marketed for many years. They serve to reduce perspiration, particularly following application to the surface of the body. Such compositions are typically considered cosmetic products, although certain countries do classify the active ingredients most commonly used in such compositions as pharmaceutical agents. The compositions are most commonly applied to the underarm regions of the human body.

The active ingredients most commonly used in antiperspirant compositions are astringent chlorohydroxide salts of aluminium and/or zirconium. These active ingredients are synthetic in origin, prepared in chemical plants and generally involving relatively advanced chemical processing steps. Such processing is not only expensive, but can also have significant environmental impact in terms of energy consumption.

Consumers are increasingly desirous of applying only "natural" ingredients and treatments to their body. Synthetic ingredients, in particular "active" ingredients, are often considered unsuitable for such application by consumers. There are a number of natural ingredients available that deliver some degree of deodorancy benefit when applied to the surface of the human body, but these ingredients are typically not capable of delivering a significant antiperspirancy benefit, i.e., they do not suppress perspiration to an extent that consumers would find desirable. Hence, there is a problem in achieving good antiperspirancy using active ingredients that are natural ingredients.

Alum salts have been disclosed as suitable for use in a range of deodorant compositions and, indeed, such products have been marketed.

US 6,139,824 (L'Oreal, 2000) discloses the use of potassium alum In water-in-oil emulsions for deodorising the body. This patent also references several other publications in which potassium alum Is used in aqueous and aqueous/ethanol solutions and in suspension sticks.

EP 1,974,716 A (Sara Lee, 2007) and WO 08/120976 (Sara Lee, 2008) disclose cosmetic compositions, for instance deodorant compositions, comprising at least partially dehydrated aluminium sulphate and a carrier liquid other than water.

Crystal Spring Ltd. offer or have offered a range of natural deodorants based upon the deodorising effect of potassium alum.

Green Bear UK Ltd. offer or have offered a crystal alum deodorant stick.

US 5,534,246 (Helen Curtis, 1996) discloses water-in-oil emulsion antiperspirant compositions in which alum salts are optional components; however no formulations containing alum salts are exemplified.

US 133,430 (John Gamgee, 1872) discloses the manufacture of a deodorising powder by mixing/grinding together aluminium sulphate (sulphate of alumina or "alum") and calcium chloride.

An objective of the present invention is to provide effective antiperspirant compositions the manufacture of which involves relatively low cost and relatively little environmental impact. In addition, the method and compositions of the invention may be seen as having good "natural" credentials, involving natural antiperspirant ingredients or at least naturally-derived antiperspirant ingredients.

A further objective of the present Invention is to provide high efficacy antiperspirant compositions.

A further objective of the present invention is to provide a highly effective method of reducing perspiration and it is a particular objective that said method does not involve the use of synthetic aluminium and/or zirconium chlorohydroxide antiperspirant actives such as aluminium chlorohydrate.

In anhydrous compositions containing astringent chlorohydroxide salts of aluminium and/or zirconium a wide range of liquids may be employed as carrier materials. These liquids are used to disperse and suspend the particulate antiperspirant chlorohydroxide salts of aluminium and/or zirconium within the composition. They may additionally be selected to provide emollient and deposit masking benefits.

When a combination of an alum salt and a water soluble calcium salt such as calcium chloride are employed in an antiperspirant composition, the carrier liquid needs to be selected with care in order to avoid issues during manufacture and subsequent storage of the formulation. If liquids having a Hildebrand Solubility Parameter (HSP) of above 18.02 (MPa)^{0.5} are employed their level of usage must be restricted. Failure to do so can result in formulations that are unstable with respect to their rheology and physical properties. Aerosol base compositions may set solid or become gritty during preparation or upon subsequent storage. Stick compositions can be formed that are undesirably soft. This is particularly the case where stearyl alcohol is used as the primary stick structurant, i.e., the structurant used in the highest weight percentage. Another problem is that stick formulations may become extremely hard upon storage, such as to make them unusable. It is hypothesised that the alum and calcium chloride react together, mediated by the relatively polar liquid.

In a first aspect of the present Invention, there is provided an anhydrous antiperspirant composition according to claim 1.

In a second aspect of the present invention, there is provided a method of reducing perspiration of the human body comprising the topical application of a composition according to the first aspect of the invention.

In a third aspect of the present invention, there is provided a method of manufacture of an anhydrous antiperspirant composition according to claim 12.

The method for reducing perspiration described herein is for reducing perspiration from the surface of the human body, in particular from the underarm areas and the feet and especially from the underarm areas, otherwise known as the axillae.

The method may generally be considered a cosmetic method and compositions used in achieving the method, cosmetic compositions. That being said, the method can be extremely effective and may be also used to treat the medical condition of extreme sweating known as hyperhidrosis.

The method typically involves topical application of a composition according to the first aspect of the invention directly to the surface of the human body. In an alternative embodiment, the composition may be applied indirectly to the surface of the human body, for example by application of said composition onto a wipe which is In turn applied to the surface of the human body.

When the composition is applied to the surface of the human body, it is hypothesised that fluids derived from the sweat glands at least partially dissolve and hence mobilise the salts, allowing them to interact and thereby deliver a good antiperspirancy benefit.

Herein, the term "dried powder" should be understood to include both crystalline and amorphous states of matter. Such powders have a water content that is reduced from that of the most hydrated natural salt of the particular salt being used. More is said concerning preferred "dried powders" in the paragraphs described preferred alum salts and preferred calcium chloride salts.

Herein, the term "dehydrated" when used with reference to a salt should be understood to refer to a salt that has a water content that is reduced from that of the most hydrated natural salt of the particular salt being used.

Herein, the term "anhydrous" should be understood to mean having less than 2% by weight of free water; "free water" being water other than the water of hydration associated with any particular component. Preferably, anhydrous compositions have less than 1% by weight free water and more preferably less than 0.5%.

Herein, the term "liquid" should be understood to refer to a state of matter at ambient temperature and pressure, by which is meant 20°C and 1 atmosphere.

Herein, the term "oil" should be understood to refer to a substance immiscible with water that is a liquid at ambient temperature and pressure.

Herein, all percentages (%) should be understood to be percentages by weight (% w/w).

It is preferred that anhydrous compositions have a total water content (including water of hydration associated with components therein) of less than 10% by weight, and more preferably less than 5%.

The term alum salt as used in the present description means aluminium sulphate (sometimes called "alum") or any double sulphate of aluminium and a univalent metal ion selected from potassium, sodium, or ammonium. It does not include alum salts that are double sulphates of a univalent metal and a trivalent metal other than aluminium, such as chromium (III) or iron (III).

Alum salts for use in the present invention are potassium alum, ammonium alum, sodium alum and aluminium sulphate. That is to say:

M(i)Al(SO₄)₂ or Al₂(SO₄)₃

wherein M(i) is K⁺, Na⁺, NH₄⁺ or mixture thereof.

Preferred alum salts are ammonium and potassium alum, in particular potassium alum.

Alum salts used in the present invention have a reduced content of water, that is to say, they are at least partially dehydrated. They may alternatively be described as dried powders (*vide supra*)*.* It has been found that use of such salts improves ease of formulation and/or leads to improved storage stability for the composition.

Potassium alum dodecahydrate has been found to be particularly difficult to formulate with calcium chloride; however, reducing its water content by 25% or greater can lead to acceptable compositions. Typical alum salls for use In present invention have a water content of less than 35% by weight. Preferred alum salts have a water content of less than 28% by weight and especially preferred alum salts have a water content of less than 20% by weight. When water is present, it is typically present as water of hydration.

The alum salt used in the present invention is typically milled to give it a reduced particle size. In preferred embodiments, the particle size distribution of the alum salt is such that its D50 is less than 75 microns, more preferably less than 50 microns, and most preferably less than 20 microns. The particle size distribution of the alum salt is preferably such that less than 5% and more preferably less, than 1% by weight of the particles have a particle size of greater than 100 microns.

The particle size distribution of the alum salt may advantageously be measured using a light scattering method on a Sympatec GmbH Helos machine with an Oasis dry powder disperser and using an air pressure of 0.5 bar (50,000 Pa) and analysing results using Frauhoffer theory.

The water soluble calcium salt used in the present invention is calcium chloride, but the chloride ion may be substituted in whole or in part by bromide, iodide, or nitrate. It should be understood that references to calcium chloride herein are generally to this material optionally substituted as described above.

Preferably, the calcium chloride is a dried powder (*vide supra*)*.* It is highly preferred that the calcium chloride has a water content of 25% or less. Suitable calcium chloride salts for such compositions include calcium chloride dihydrate and anhydrous calcium chloride, with anhydrous calcium chloride being preferred. It should be noted, however, than anhydrous calcium chloride as obtained from some suppliers can include up to about 14% by weight of water of hydration.

The calcium chloride used in the present invention is typically milled to give it a reduced particle size. In preferred embodiments, the particle size distribution of the calcium chloride Is such that its D50 is less than 100 microns and preferably less than 75 microns. The particle size distribution of the calcium chloride is preferably such that less than 5% and more preferably less than 1% by weight of the particles have a particle size of greater than 100 microns.

The particle size distribution of the alum salt may advantageously be measured using a light scattering method on a Sympatec GmbH Helos machine with an Oasis dry powder disperser and using an air pressure of 0.5 bar (50,000 Pa) and analysing results using Frauhoffer theory.

Central to the present invention is the timely triggering of the following chemical reaction:

KAl(SO₄)₂ + 2CaCl₂ → 2CaSO₄↓ + KCl + AlCl₃

Or

Al₂(SO₄)₃ + 3CaCl₂ → 3CaSO₄↓ + 2AlCl₃

In the top equation, the potassium ion (K⁺) may be substituted by sodium (Na⁺) or ammonium (NH₄⁺).

In both equations, the chloride ion used may be substituted by bromide, iodide, or nitrate. Thus, the calcium chloride could equally well be calcium bromide, iodide, nitrate or any mixture thereof.

The stoichiometry of the above equations requires one mole of alum to two moles of calcium chloride in the first and one mole of alum to three moles of calcium chloride in the second. These equations set the basis for the preferred ratios of these components in compositions according to the invention. In such compositions, It is preferred that the molar quantity of calcium chloride exceeds the molar quantity of alum salt. It is also preferred that the quantity of calcium chloride at least matches that stoichiometrically required by the above equations, relative to the amount and type of alum present. This means that it is preferred that the molar ratio of calcium chloride to alum salt is at least 2:1.

In compositions comprising calcium chloride and sodium, potassium or ammonium alum as the major alum salt present, the molar ratio of calcium chloride to alum salt is preferably from 1:1 to 5:1, more preferably from 3:2 to 3:1, and most preferably about 2:1.

In compositions comprising calcium chloride and aluminium sulphate as the major alum salt present, the molar ratio of calcium chloride to alum salt is preferably from 2:1 to 6:1, more preferably from 5:2 to 4:1, and most preferably about 3:1.

It is important to the present invention that the reaction indicated above only occurs to a minimal extent before the components are delivered to the surface of the human skin. Premature reaction results in a physical state of matter which tends not to deliver the desired benefits; indeed, it is commonly extremely difficult to even apply said matter to the desired location.

The chemical reaction involved in the present invention may only occur when the ions making up the reactants have sufficient mobility. In certain preferred embodiments of the present invention, this mobility typically arises when the reactants dissolve in aqueous body fluids found on the surface of the human body.

Magnesium chloride was found to be ineffective when used instead of calcium chloride because of the much greater water solubility of magnesium sulphate compared with calcium sulphate.

A third essential component of the invention is a liquid carrier material comprising 80% or more by weight of oils having a HSP of 18.02 (MPa)^{0.5} or less. Preferably, the liquid carrier material comprises 85% or more of such oils and more preferably 90% or more.

It will be understood that the liquid carrier material may only comprise liquids having a HSP of greater than 18.02 (MPa)^{0.5} in an amount of less than 20%, preferably less than 10%, and more preferably less than 5% by weight.

The present Inventors have found that for stick or soft solid compositions comprising a structurant, the preferred levels of oils having a HSP of 18.02 (MPa)^{0.5} or less is somewhat higher. Thus, in a particular aspect of the invention, there is provided a stick or soft solid composition comprising a dehydrated alum salt, a water soluble calcium salt, and a liquid carrier material comprising 85% or more by weight of oils having a HSP of 18.02 (MPa)^{0.5} or less. In such compositions, the liquid carrier material preferably comprises 90% or more and more preferably 95% or more by weight of oils having a HSP of 18.02 (MPa)^{0.5} or less.

The liquid carrier material should be considered to include all the liquid components of the composition, including liquid fragrance components, when present.

HSPs for many cosmetic liquids can be obtained from the literature, such as from C. D. Vaughan, J. Soc. Cosmet. Chem. (1985), 36, 319-333 or from Table 1 in US 2010/0047296 (Henkel). For liquids where literature values have not been reported they may be calculated using methods described in the above reference or the methods described by S. W. van Krevelen in Properties of Polymers, p200-225, Elsevier, (1990).

Suitable oils having a HSP of 18.02 (MPa)^{0.5} or less are triethylhexanoin, isopropyl myristate, isopropyl palmitate, diisopropyl adipate, hexadecane, C12-15 alkyl benzoate, dioctyl ether, white mineral oil, dimethicone fluid, phenyl trimethicone and cyclomethicones such as cyclopentasiloxane. In preferred embodiments, the liquid carrier material comprises 80% or more by weight of oils selected from this list.

The liquid carrier material comprising 80% or more by weight of oils having a HSP of 18.02 (MPa)^{0.5} or less preferably comprises oils having a HSP of 16.36 (MPa)^{0.5} or less. In preferred embodiments, the liquid carrier material comprises 80% or more by weight of oils having a HSP of 16.36 (MPa)^{0.5} or less.

Suitable oils, having a HSP of 16.36 (MPa)^{0.5} or less are hexadecane, C12-15 alkyl benzoate, isopropyl palmitate, dioctyl ether, white mineral oil, dimethicone fluid, phenyl trimethicone and cyclomethicones such as cyclopentasiloxane. In particularly preferred embodiments, the liquid carrier material comprises 85% or more by weight of oils selected from this list.

Oils with a HSP of greater than 18.02 (MPa)^{0.5} that can be included include ethers, such as PPG-14 butyl ether, and fatty alcohols, such as octyl dodecanol and isocetyl alcohol.

Preferred components of the liquid carrier material also perform an addition function; particularly preferred functions being to act as emollients and/or masking liquids.

Preferred components of the liquid carrier material are anhydrous, as described hereinabove. Preferably, they contain less than 2%, more preferably less than 1% and most preferably less than 0.5% by weight free water.

The liquid carrier material is preferably included in the composition at a level of 1-90%, more preferably at from 10 to 80% and most preferably at from 20 to 70% by weight of the composition, excluding any propellant that might also be present.

Oils having a HSP of 18.02 (MPa)^{0.5} or less are preferably included in the composition at a level of 1-90%, more preferably at from 10 to 80% and most preferably at from 20 to 70% by weight of the composition, excluding any propellant that might also be present.

Other components may also be included in compositions used in accordance with the invention.

Additional antiperspirant actives may also be included.

The total amount of antiperspirant actives, including dehydrated alum salt and calcium chloride, incorporated in a composition is preferably from 0.5-50%, particularly from 1 to 30% and especially from 2% to 26% of the weight of the composition.

The proportion of solid antiperspirant salt in a suspension composition normally includes the weight of any water of hydration and any complexing agent that may also be present in the solid active.

Additional deodorant actives may also be included. When employed, the level of incorporation is preferably from 0.01% to 3% and more preferably from 0.03% to 0.5% by weight. Preferred deodorant actives are those that are more efficacious than simple alcohols such as ethanol. Examples include quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred are polyhexamethylene biguanide salts (also known as polyaminopropyl biguanide salts), an example being Cosmocil CQ available from Arch Chemicals; 2',4,4'-trichloro,2-hydroxy-diphenyl ether (triclosan); and 3,7,11-trimethyldodeca-2,6,10-trienol (farnesol).

Other components particular to the type of composition in which the invention is used may also be Included. Types of composition in which the invention may be used include, non-exclusively, sticks, soft solids, aerosols, and roll-ons.

Stick or soft solid compositions typically comprise one or more structurants or gellants, which serves to thicken the composition. Such thickeners, referred to as structurant systems, may be selected from those known in the art for such purpose. The present inventors have found the choice of structurants to be of particular importance when the alum salt and calcium chloride are included in the same composition. In such compositions, it has been found that particularly suitable structurant systems comprise:
1. stearyl alcohol as the major component, preferably in the presence of lesser amounts of polyethylene wax and hydrogenated castor oil; or
2. polyethylene wax as the major component, preferably in the presence of lesser amount of hydrogenated castor oil.

In general, structurant and gellants suitable for use in compositions according to the present invention may be classed as waxes or non-polymeric fibre-forming gellants.

"Waxes" may be defined as water-insoluble materials that are solid at 30°C and preferably also at 40°C. They may be selected from hydrocarbons, linear fatty alcohols, silicone polymers, esters waxes or mixtures thereof.

Examples of hydrocarbon waxes include paraffin wax, ozakerite, microcrystalline wax and polyethylene wax, the last named desirably having an average molecular weight of from 300 to 600 and advantageously from 350 to 525.

Linear fatty alcohols commonly contain from 14 to 40 carbon atoms and often from 16 to 24. In practice, most contain an even number of carbon atoms and many comprise a mixture of compounds, even those that are nominally a single one such as stearyl alcohol.

Silicone polymer waxes typically satisfy the empirical formula:-

1. R-(SiMe₂-O-)ₓ-SiMe₂R

in which x is at least 10, preferably 10 to 50 and R represents an alkyl group containing at least 20 carbons, preferably 25 to 40 carbons, and particularly having an average linear chain length of at least 30 carbons; or

2. Y-(SiMe₂-O-)_{y}(Si[OR']Me-O-)_{z}-Y'

in which Y represents SiMe₂-O, Y' SiMe₂, R' an alkyl of at least 15 carbons preferably 18 to 22 such as stearyl, y and z are both integers, totalling preferably from 10 to 50.

Examples of ester waxes include esters of C₁₆-C₂₂ fatty acids with glycerol or ethylene glycol, which can be isolated from natural products or more conveniently synthesised from the respective aliphatic alcohol and carboxylic acid.

"Non-polymeric fibre-forming gellants" are capable of being dissolved in a water-immiscible blend of oils at elevated temperature and on cooling precipitating out to form a network of very thin strands that are typically no more than a few molecules wide. One particularly effective category of such thickeners comprises N-acyl aminoacid amides and in particular linear and branched N-acyl glutamic acid dialkylamides, such as in particular N-lauroyl glutamic acid di n-butylamide and N-ethylhexanoyl glutamic acid di n-butylamide and especially mixtures thereof. Such amido gellants can be employed in anhydrous compositions according to the present invention, if desired, with 12-hydroxystearic acid.

Other such non-polymeric fibre-forming gellants Include 12-hydroxystearic acid amides, and amide derivatives of di- and tri-basic carboxylic acids as set forth in WO 98/27954, including notably alkyl N,N'dialkyl succinamides.

Further suitable structuring systems comprising non-polymeric fibre-forming gellants of this type are described in US 6,410,003, US 7,332,153, US 6,410,001, US 6,321,841, and US 6,248,312.

The structurant or gellant is often employed in the stick or soft solid composition at a concentration of from 1.5 to 30%. When a non-polymeric fibre-forming gellants is employed as the major component of the structuring system, its concentration is typically in the range of from 1.5 to 7.5% by weight for amido gellants or mixtures of them and for 5 to 15% for ester or sterol gellants. When a wax is employed as the major component of the structuring system, its concentration is usually selected in the range of from 10 to 30% by weight, and particularly from 12 to 24% by weight.

Other types of structurant or gellant disclosed in the prior art may alternatively be employed.

Aerosol compositions are comprised of a propellant and a base. An aerosol "base" composition is considered to be all the components of the aerosol composition minus the propellant.

It should be noted the propellants, even when liquefied, are not liquids according to the present invention, as they have boiling points below 20°C at atmospheric pressure.

Typically, the aerosol propellant is a liquefied hydrocarbon or halogenated hydrocarbon gases (particularly fluorinated hydrocarbons such as 1,1-difluoroethane and/or 1-trifluoro-2-fluoroethane) that have a boiling point of below 10°C and especially those with a boiling point below 0°C. It is especially preferred to employ liquefied hydrocarbon gases, and especially C₃ to C₆ hydrocarbons, including propane, butane, isobutane, pentane and isopentane and mixtures of two or more thereof. Preferred propellants are isobutane, isobutane/propane, butane/propane and mixtures of propane, isobutane and butane.

Other propellants that can be contemplated include alkyl ethers, such as dimethyl ether or compressed non-reactive gasses such air, nitrogen or carbon dioxide.

The propellant is typically the major component of aerosol compositions, often comprising from 30 to 99% weight and preferably comprising from 50 to 95% by weight.

In certain preferred embodiments, aerosol compositions may also comprise a suspending agent, for example, a hydrophobically modified clay, such as disteardimonium hectorite (Bentone 38V), *ex* Elementis, typically at from 0.1 to 1.5% by weight.

Propylene carbonate may also be advantageously employed in aerosol compositions used in accordance with the present invention, typically at from 0.001 to 0.1% by weight.

Roll-on compositions suitable for use in accordance with the invention are typically suspension products, in particular suspensions in one or more anhydrous liquid carrier materials (*vide supra*), hydrophobic liquid carrier materials being preferred.

Roll-on compositions preferably comprise a suspending agent, for example, a hydrophobically modified clay, such as disteardimonium hectorite (Bentone 38V), *ex* Elementis, typically at from 0.5 to 3% by weight.

Roll-on compositions preferably comprise a particulate sensory modifier, for example finely divided clay such as Aerosil 200, *ex* Evonik Degussa, typically at from 0.01 to 0.5% by weight. Other particulate sensory modifiers include particulate polyethylene (e.g. Acumist B18), talc and titanium dioxide.

In certain compositions, emulsifiers that are perfume solubilisers and/or wash-off agents are preferred additional components. Examples of the former include PEG-hydrogenated castor oil, available from BASF in the Cremaphor RH and CO ranges, preferably present at up to 1.5% by weight, more preferably 0.3 to 0.7% by weight. Examples of the latter include poly(oxyethylene) ethers.

In many embodiments of the invention, fragrance is a desirable additional component. Suitable materials Include conventional perfumes, such as perfume oils and also include so-called deo-perfumes, as described In EP 545,556, for example. Levels of incorporation are preferably up to 5% by weight, particularly from 0.1% to 3.5% by weight, and especially from 0.5% to 2.5% by weight. The fragrance may also be added in an encapsulated form, release being triggered post-application by hydrolysis or shear on the surface of the human body.

Further additional components that may also be included are colourants and preservatives at a conventional level, for example C₁-C₃ alkyl parabens.

In some compositions water miscible emollients such as polyethylenglycols (eg PEG 400) can be included, typically at levels up to 2% of the total composition.

The method of manufacture of compositions according to the invention comprises an alum salt being reduced in water content prior to mixing with the calcium chloride and a liquid carrier material comprising 85% or more by weight of oils having a Hildebrand solubility parameter of 18.02 (MPa)^{0.5} or less. In such methods, the alum salt is preferably reduced in water content to less than 35%, more preferably less than 28% and most preferably less than 20% by weight.

### Examples

The following examples illustrate certain specific embodiments of the invention and do not limit the scope of the invention. Examples according to the Invention are indicated by numbers and comparative examples are indicated by letter.

All amounts are percentages by weight, unless otherwise indicated.

The components/ingredients used in the current study are detailed below.
(1) Cyclopentasiloxane, DC245. *ex* Dow Corning. [HSP = 11.80 (MPa)^{0.5}].
(2) C12-15 alkyl benzoate, Finsolv TN, *ex* Finetex. [HSP=15.60 (MPa)^{0.5}].
(3) PPG-14 butyl ether, Fluid AP, *ex* Amercol or Ucon. [HSP = 18.61 (Mpa)^{0.5}]
(4) Dimethicone fluid (50cs), Xiameter PMX-200, ex Dow Corning. [HSP = 12.07 (MPa)^{0.5}].
(5) White mineral oil, Silkolene Sirius M70, ex Fuchs. [HSP = 14.50 (MPa)^{0.5}].
(6) Hexadecane, ex Sigma-Aldrich. [HSP_= 16.36 (MPa)^{0.5}].
(7) Isopropyl myristate, Crodamol IPM-LQ, ex Croda. [HSP = 16.40 (MPa)^{0.5}].
(8) Triethylhexanoin, Crodamol GTEH-LQ(MV), ex Croda. [HSP = 18.02 (MPa)^{0.5}].
(9) Octyl dodecanol, Eutanol G, ex Cognis, [HSP = 18.25 (MPa)^{0.5}].
(10) PEG 8, Polyglykol 400. ex Clariant, [HSP = 23.20 (MPa)^{0.5}].
(11) Stearyl alcohol, Lanette C18 Deo, *ex* Cognis.
(12) Polyethylene wax, Performalene 400, molecular weight ca. 400, *ex* Alfa Chemicals.
(13) Hydrogenated castor oil, Castor Wax MP80, *ex* Caschem.
(14) Potassium Alum ex Sigma-Aldrich dried at 65°C [to 15.5% H₂O] and jet milled to give a particle size (D50) of 17 micron.
(15) Potassium Alum *ex* Sigma-Aldrich dried at 200°C [to 3% H₂O) and Jet milled to give a particle size (D50) of 6 micron.
(16) Anhydrous Calcium Chloride - Less than 4% water (by weight), *ex* Sigma-Aldrich, jet milled to give a particle size (D50) of 14 micron.
(17) Anhydrous Calcium Chloride - 7 % water (by weight), *ex* Sigma-Aldrich, jet milled to give a particle size (D50) of between 10-20 micron.
(18) Disteardimonium hectorite, Bentone 38V, *ex* Elementis.
(19) CAP40, *ex* HARP.
(20) Propylene carbonate.
(21) Aluminium sulphate, Tai-ace S100, *ex* Taimei Chemicals Co Ltd., average particle size 8-10 microns, water content less than 1.5% by weight.
(22) Calcium chloride dihydrate, *ex* Sigma-Aldrich, found have particles of predominately less than 100 microns by optical microscopy following processing.

In a first series of experiments, compositions according to the general formula indicated in Table 1 were prepared using the liquids listed in Table 2. Table 2 also gives details of the re-dispersibility characteristics of the compositions after storage at 45°C for 4 weeks.

**Table 1 - Liquid Composition**

| **Component** | **Amount (%)** |
|---|---|
| Dehydrated alum (14) | **20** |
| Calcium chloride (16) | **14** |
| **Liquid carrier (see Table 2)** | **66** |

**Table 2 - Liquid Compositions**

| **Example** | **Liquid carrier** | **HSP - (MPa)^{0.5}** | **Re-dispersibility** |
|---|---|---|---|
| **1** | Dimethicone fluid (50cs) (4) | 12.07 | **Easy** |
| **2** | White mineral oil (5) | 14.50 | **Difficult** |
| **3** | C12-15 alkyl benzoate (2) | 15.60 | **Difficult** |
| **4** | Hexadecane (6) | 16.36 | **Difficult** |
| **5** | Isopropyl myristate (7) | 16.40 | **Difficult** |
| **6** | Triethylhexanoin (8) | 18.02 | **Difficult** |
| **A** | Octyl dodecanol (9) | 18.25 | **Impossible** |
| **B** | PPG-14-butyl ether (3) | 18.61 | **Impossible** |
| **C** | **PEG 8 (10)** | **23.20** | **Impossible** |

With the liquids having HSP values of greater than 18.02 (MPa)^{0.5}, it was found that the alum and calcium chloride had formed a hard compact layer which was impossible to re-disperse. It is hypothesised that the alum and calcium chloride had reacted together, mediated by the relatively polar liquid. With the oils having HSP values of 18.02 (MPa)^{0.5} or less, it was found that the alum and calcium chloride were re-dispersible to a greater or lesser extent. When the solids were "difficult" to re-disperse, prolonged shaking was required to break up the caked powder; however, a suspension of fine solids ultimately resulted.

In a second series of experiments, compositions according to the general formula Indicated in Table 1 were prepared using mixtures of oils (making up 66% of the composition in total), as Indicated In Table 3. Table 3 also gives details of the re-dispersibility characteristics of the compositions after storage at 45°C for 4 weeks.

**Table 3 - Further Liquid Compositions**

| **Example** | Liquid carrier; Relative amount | | | | Re-dispersibility |
|---|---|---|---|---|---|
| Oil: | (1) | (2) | (8) | (3) | |
| **7** | 50 | 50 | 0 | 0 | Easy |
| **8** | 38 | 38 | 24 | 0 | Difficult |
| **9** | 25 | 25 | 50 | 0 | Difficult |
| **10** | 47 | 47 | 0 | 6 | Difficult |
| **D** | 38 | 38 | 0 | 24 | Sticky |
| **E** | 25 | 25 | 0 | 50 | Impossible |

The results show that in mixtures of cyclopentasiloxane (1), C12-15 alkyl benzoate (2) and triethylhexanoin (8), the alum and calcium chloride could be redispersed fully. Full re-dispersion was also possible when a low level of PPG-14 butyl ether (3) was included (Example 10). At higher levels of PPG-14 butyl ether (3) the solids formed a "sticky" aggregate that could not be fully dispersed to a fine powder, or were "impossible" to disperse.

Antiperspirant aerosol base compositions as indicated in Table 4 were prepared as follows. The oil or oils [components (1) (2) (3) and (8)] were blended at ambient temperature with the suspending agent [component (18)], followed by the propylene carbonate and fragrance, each being added with shear. Salts selected from components (14) to (17) as indicated were then added (calcium chloride first) and well dispersed into the mixture. The resulting base compositions were then assessed for consistency and stability.

**Table 4 - Aerosol Base Compositions:**

| **Example:** | **F** | **11** | **12** | **13** | **14** | **15** |
|---|---|---|---|---|---|---|
| **Component:** | | | | | | |
| Oil (1) | 45.30 | 60.68 | 63.76 | 45.30 | 48.38 | 44.77 |
| Oil (2) | -- | -- | -- | 15.38 | 15.38 | -- |
| Oil (3) | 15.38 | -- | -- | -- | -- | -- |
| Oil (8) | -- | -- | -- | -- | -- | 14.91 |
| Propylene carbonate (20) | 0.08 | 0.08 | 0.08 | 0.08 | 0.08 | 0.08 |
| Fragrance | 4.62 | 4.62 | 4.62 | 4.62 | 4.62 | 4.62 |
| Alum (14) | 18.00 | 18.00 | -- | 18.00 | -- | 18.00 |
| Alum (15) | -- | -- | 14.92 | -- | 14.92 | |
| CaCl₂ (16) | 12.77 | 12.77 | 12.77 | 12.77 | 12.77 | |
| CaCl₂ (17) | | | | | | 13.77 |
| Suspending agent (18) | 3.85 | 3.85 | 3.85 | 3.85 | 3.85 | 3.85 |

Each of the examples according to the invention was a stable, pourable base which remained pourable for at least three days at ambient temperature. Comparative Example F, on the other hand, became hot immediately after preparation and proceeded to completely solidify within one hour, making it unusable.

The aerosol base composition Example 13 was subsequently used to form a full aerosol composition. The base was placed into an aerosol cans which were closed with a standard valve and valve cup and the liquefied propellant (component (19)) then added. The weight ratio of base to propellant was 13:87.

The antiperspirancy efficacy of the aerosol composition made from Example 13 was compared with that of a non-antiperspirant body spray control. Test operators applied the test composition (a two second spray - equivalent to approximately 2g application) to one axilla and a similar amount of the body spray control to the other axilla of each panellist. This was done once each day for three days. After the third application, panellists were requested not to wash under their arms for the following 24 hours.

24 hours after the third and final product application, the panellists were induced to sweat in a hot-room at 40°C (±2°C) and 40% (±5%) relative humidity, for 40 minutes. After this period, the panellists left the hot-room and their axillae were carefully wiped dry. Pre-weighed cotton pads were then applied to each axilla of each panellist and the panellists re-entered the hot-room for a further 20 minutes. Following this period, the pads were removed and re-weighed, enabling the weight of sweat generated to be calculated.

The sweat weight reduction (SWR) for each panellist was calculated as a percentage (% SWR) and the mean % SWR was calculated according to the method described by Murphy and Levine in "Analysis of Antiperspirant Efficacy Results", J. Soc. Cosmetic Chemists, 1991(May), 42, 167-197.
In this test Example 3 was found to give a 23% greater SWR than the control.

Further antiperspirant aerosol base compositions indicated in Table 5 were prepared in the same manner as those in Table 4.

**Table 5: Further Aerosol Base Compositions**

| **Example:** | **16** | **17** | **18** | **19** | **20** | **21** |
|---|---|---|---|---|---|---|
| **Component:** | | | | | | |
| Oil (1) | 57.87 | 55.90 | 55.90 | 52.14 | 57.87 | 55.90 |
| Oil (3) | 1.79 | 3.76 | | | | |
| Oil (9) | | | 3.76 | 7.52 | | |
| PEG 8 (10) | | | | | 1.79 | 3.76 |
| Propylene carbonate (20) | 0.08 | 0.08 | 0.08 | 0.08 | 0.08 | 0.08 |
| Fragrance | 4.62 | 4.62 | 4.62 | 4.62 | 4.62 | 4.62 |
| Alum (14) | 18.02 | 18.02 | 18.02 | 18.02 | 18.02 | 18.02 |
| CaCl₂ (17) | 13.77 | 13.77 | 13.77 | 13.77 | 13.77 | 13.77 |
| Suspending agent (18) | 3.85 | 3.85 | 3.85 | 3.85 | 3.85 | 3.85 |

Each of the compositions was a stable, pourable base which remained pourable for at least three days at ambient temperature, despite each one containing low levels of liquid carrier materials having a HSP of greater than 18.02 (MPa)^{0.5}. Example 19 is notable, octyl dodecanol (9) (an oil having a HSP of 18.25 (MPa)^{0.5}) comprising 11.7% of the liquid carrier material of this composition. Example 21 is also notable, PEG 8 (10) (a liquid having a HSP of 23.2 (MPa)^{0.5}) comprising 5.8% of the liquid carrier material of this composition. Compositions comprising water miscible humectants, of which PEG 8 is one, are particularly desirable because of their humectancy properties.

The stick compositions indicated in Table 6 were prepared as follows. The oils [components (1) (2) (3) and (8)] were blended together at 90°C and the waxes [components (11) to (13)] were melted in with stirring. When the waxes were fully melted, the mixtures were cooled to 75-85°C and salts selected from components (14) to (17) as Indicated were added (calcium chloride first) and well dispersed into the mixture. The mixtures cooled to about 62°C and poured into stick barrels.

**Table 6 - Stick Compositions**

| **Example:** | **22** | **23** | **24** | **25** | **26** | **27** |
|---|---|---|---|---|---|---|
| **Component:** | | | | | | |
| Oil (1) | 39.5 | 41.1 | 38.87 | 50.5 | 39.5 | 45.73 |
| Oil (2) | 11.0 | 11.0 | 20.17 | 11.0 | 22.0 | -- |
| Oil (3) | 11.0 | 11.0 | 1.83 | -- | -- | -- |
| Oil (8) | -- | -- | -- | -- | -- | 15.25 |
| Wax (11) | 18.0 | 18.0 | 18.0 | 18.0 | 18.0 | 18.0 |
| Wax (12) | 1.0 | 1.0 | 1.0 | 1.0 | 1.0 | 1.0 |
| Wax (13) | 3.5 | 3.5 | 3.5 | 3.5 | 3.5 | 3.5 |
| Alum (14) | 9.35 | -- | 9.35 | 9.35 | 9.35 | 9.35 |
| Alum (15) | -- | 7.75 | -- | -- | -- | -- |
| CaCl₂ (16) | 6.65 | 6.65 | | 6.65 | 6.65 | |
| CaCl₂ (17) | | | 7.16 | | | 7.16 |

Examples 22 was used in an antiperspirancy efficacy test similar to the one used to test the full aerosol composition prepared from Example 13. A significant SWR was obtained using Example 22 (dosed at 0.3 g per application).

Scale-up studies revealed that Examples 22 and 23 were inferior to Examples 24 to 27, being less robust to processing conditions. Examples 22 and 23 became unacceptably soft when made at large (3 kg) scale, being intolerant of being processed at 80°C or higher for prolonged periods. Evidence of the superiority of Examples 24 to 27 over versions of Examples 22 and 23 processed at 85°C for two hours (labelled 22A and 23A, respectively) is given in Table 7 as penetrometer values. It will be seen that Examples 22A and 23A were very soft, having penetrometer values of greater than 15 mm. By contrast, Examples 24 to 27 (also processed at 85°C for two hours), each had acceptable penetrometer values of 7.3 mm to 9.6 mm.

**Table 7**

| **Example:** | **22A** | **23A** | **24** | **25** | **26** | **27** |
|---|---|---|---|---|---|---|
| Appearance | Mushy | Mushy | Hard | Hard | Hard | **Hard** |
| **Hardness (mm)** | **>15** | **>15** | **7.3** | **8.9** | **9.6** | **7.5** |

The penetrometer used to make the measurements Indicated in Table 7 was a lab plant PNT penetrometer equipped with a Seta wax needle (weight 2.5 g) having a cone angle at the point of the needle of 9°10' +/- 15'. Each composition tested had a flat upper surface onto which the needle was lowered and penetration hardness was measured by allowing the needle with its holder to drop under the combined weight of needle and holder (50 g) for a period of five seconds. The test was carried out at six points on each sample and the results then averaged.

The Examples indicated in Table 8 were prepared as follows. The oils were blended together at 95°C and the waxes were melted in with stirring at 90-95°C. When the waxes were fully melted, the mixtures were cooled to 85°C and salts selected as indicated were added (calcium chloride first) and well dispersed into the mixture. The mixtures were cooled to about 75°C and poured into stick barrels.

**Table 8 - Further Stick Compositions**

| **Example:** | **28** | **29** | **30** | **31** | **32** | **33** |
|---|---|---|---|---|---|---|
| **Component:** | | | | | | |
| Oil (1) | To 100 | To 100 | To 100 | To 100 | To 100 | To 100 |
| Oil (2) | 30.0 | 30.0 | 30.0 | 30.0 | 30.0 | 30.0 |
| Wax (12) | 15.0 | 15.0 | 15.0 | 15.0 | 15.0 | 15.0 |
| Wax (13) | 2.0 | 2.0 | 2.0 | 2.0 | 2.0 | 2.0 |
| Alum (14) | 9.4 | -- | -- | -- | 9.4 | 14.1 |
| Alum (15) | -- | 7.8 | -- | 7.8 | -- | -- |
| Aluminium sulphate (21) | -- | -- | 5.1 | -- | -- | -- |
| CaCl₂ (16) | 6.7 | 6.7 | 5.0 | -- | -- | 10.0 |
| CaCl₂ (22) | -- | -- | -- | 8.8 | 8.8 | -- |

All of these stick compositions had satisfactory hardness and Example 28 was found to give a significant SWR when tested using methodology similar to that used to test Examples 13 and 22.

## Claims

1. An anhydrous antiperspirant composition having less than 2% by weight of free water comprising a dehydrated alum salt (by which is meant aluminium sulphate or any double sulphate of aluminium and a univalent metal selected from potassium, sodium, or ammonium), a water soluble calcium salt, and a liquid carrier material comprising 80% or more by weight of oils having a Hildebrand solubility parameter of 18.02 (MPa)^{0.5} or less and not including synthetic aluminium and/or zirconium chlorohydroxide antiperspirant actives such as aluminium chlorohydrate, wherein the water soluble calcium salt is calcium chloride and wherein the chloride in the calcium chloride may be substituted in whole or in part by bromide, iodide or nitrate.

2. An antiperspirant product according to claim 1, wherein the alum salt is potassium aluminium sulphate.

3. An antiperspirant composition according to any of the preceding claims having a molar ratio of calcium chloride to alum salt that is greater than 1:1.

4. An antiperspirant product according to claim 3, wherein the molar ratio of calcium chloride to alum salt that is at least 2:1.

5. An antiperspirant composition according to any of the preceding claims, wherein the alum salt has a water content of less than 35% by weight, preferably less than 28% by weight and more preferably less than 20% by weight.

6. An antiperspirant composition according to any of the preceding claims, wherein the water soluble calcium salt has a water content of less than 15% and preferably less than 8%.

7. An antiperspirant composition according to any of the preceding claims, wherein a liquid carrier material comprises 85% or more by weight of oils having a Hildebrand solubility parameter of 18.02 (MPa)^{0.5} or less.

8. An antiperspirant composition according to claim 7, wherein a liquid carrier material comprises 90% or more by weight of oils having a Hildebrand solubility parameter of 18.02 (MPa)^{0.5} or less.

9. An antiperspirant composition according to any of the preceding claims, that is an aerosol composition comprising a propellant.

10. An antiperspirant composition according to any of the preceding claims, that is a stick or soft solid composition comprising a structurant.

11. A method of reducing perspiration of the human body comprising the topical application of a composition according to any of the preceding claims.

12. A method of manufacture of an anhydrous antiperspirant composition having less than 2% by weight of free water comprising the reduction in water content of an alum salt (by which is meant aluminium sulphate or any double sulphate of aluminium and a univalent metal selected from potassium, sodium, or ammonium) and the combination of the resulting dehydrated alum salt with a water soluble calcium salt and a liquid carrier material comprising 80% or more by weight of oils having a Hildebrand solubility parameter of 18.02 (MPa)^{0.5} or less and the exclusion of synthetic aluminium and/or zirconium chlorohydroxide antiperspirant actives such as aluminium chlorohydrate.

13. A method according to claim 12, wherein the alum salt is reduced in water content to less than 28% by weight prior to its combination with the water soluble calcium salt and a liquid carrier material.

## Patentansprüche

1. Wasserfreie schweißhemmende Zusammensetzung, die weniger als 2 Gew.-% ungebundenes Wasser aufweist, die ein dehydratisiertes Alaunsalz (womit Aluminiumsulfat oder irgendein Doppelsulfat von Aluminium und einem einwertigen Metall, das aus Kalium, Natrium ausgewählt ist, oder Ammonium gemeint ist), ein wasserlösliches Calciumsalz und ein flüssiges Trägermaterial aufweist, das 80 Gew.-% oder mehr Öle mit einem Löslichkeitsparameter nach Hildebrand von 18,02 (MPa)^{0,5} oder weniger aufweist, und keine synthetischen schweißhemmenden Wirkstoffe in Form von Aluminium- und/oder Zirconiumchlorhydroxid, wie Aluminiumchlorhydrat, aufweist, wobei das wasserlösliche Calciumsalz Calciumchlorid ist und wobei das Chlorid in dem Calciumchlorid gänzlich oder zum Teil durch Bromid, Jod oder Nitrat ersetzt werden kann.

2. Schweißhemmendes Produkt nach Anspruch 1,
wobei das Alaunsalz Kaliumaluminiumsulfat ist.

3. Schweißhemmende Zusammensetzung nach einem der vorstehenden Ansprüche, die ein Molverhältnis zwischen Calciumchlorid und Alaunsalz aufweist, das mehr als 1:1 beträgt.

4. Schweißhemmendes Produkt nach Anspruch 3,
wobei das Molverhältnis zwischen Calciumchlorid und Alaunsalz mindestens 2:1 beträgt.

5. Schweißhemmende Zusammensetzung nach einem der vorstehenden Ansprüche,
wobei das Alaunsalz einen Wassergehalt von weniger als 35 Gew.-%, vorzugsweise weniger als 28 Gew.-% und stärker bevorzugt weniger als 20 Gew.-% aufweist.

6. Schweißhemmende Zusammensetzung nach einem der vorstehenden Ansprüche,
wobei das wasserlösliche Calciumsalz einen Wassergehalt von weniger als 15 % und vorzugsweise von weniger als 8 % aufweist.

7. Schweißhemmende Zusammensetzung nach einem der vorstehenden Ansprüche, wobei das flüssige Trägermaterial 85 Gew.-% oder mehr Öle mit einem Löslichkeitsparameter nach Hildebrand von 18,02 (MPa)^{0,5} oder weniger aufweist.

8. Schweißhemmende Zusammensetzung nach Anspruch 7,
wobei das flüssige Trägermaterial 90 Gew.-% oder mehr Öle mit einem Löslichkeitsparameter nach Hildebrand von 18,02 (MPa)^{0,5} oder weniger aufweist.

9. Schweißhemmende Zusammensetzung nach einem der vorstehenden Ansprüche, die eine Aerosolzusammensetzung ist, die ein Treibmittel aufweist.

10. Schweißhemmende Zusammensetzung nach einem der vorstehenden Ansprüche, die ein Stift oder eine weiche feste Zusammensetzung ist, die ein strukturbildendes Mittel aufweist.

11. Nicht-therapeutisches Verfahren zur Verminderung der Schweißbildung des menschlichen Körpers,
das die topische Anwendung einer Zusammensetzung nach einem der vorstehenden Ansprüche aufweist.

12. Verfahren zum Herstellen einer wasserfreien schweißhemmenden Zusammensetzung, die weniger als 2 Gew.-% ungebundenes Wasser aufweist, das Folgendes aufweist: Verringern des Wassergehalts eines Alaunsalzes (womit Aluminiumsulfat oder irgendein Doppelsulfat von Aluminium und einem einwertigen Metall, das aus Kalium, Natrium ausgewählt ist, oder Ammonium gemeint ist) und Kombinieren des entstandenen dehydratisierten Alaunsalzes mit einem wasserlöslichen Calciumsalz und einem flüssigen Trägermaterial, das 80 Gew.-% oder mehr Öle mit einem Löslichkeitsparameter nach Hildebrand von 18,02 (MPa)^{0,5} oder weniger aufweist, und Ausschließen von synthetischen schweißhemmenden Wirkstoffen in Form von Aluminium- und/oder Zirconiumchlorhydroxid, wie Aluminiumchlorhydrat, wobei das wasserlösliche Calciumsalz Calciumchlorid ist und wobei das Chlorid in dem Calciumchlorid gänzlich oder zum Teil durch Bromid, Jod oder Nitrat ersetzt werden kann.

13. Verfahren nach Anspruch 12,
wobei der Wassergehalt des Alaunsalzes vor seiner Kombination mit dem wasserlöslichen Calciumsalz und einem flüssigen Trägermaterial auf weniger als 28 Gew.-% verringert wird.

## Revendications

1. Composition d'antiperspirant anhydre présentant moins de 2 % en poids d'eau libre comprenant un sel d'alun déshydraté (par lequel on indique du sulfate d'aluminium ou tout sulfate double d'aluminium et d'un métal univalent choisi parmi le potassium, le sodium, ou l'ammonium), un sel de calcium soluble dans l'eau, et un matériau de support liquide comprenant 80 % en poids ou plus d'huiles présentant un paramètre de solubilité Hildebrand de 18,02 (MPa)^{0,5} ou inférieur et ne comprenant pas d'aluminium synthétique et/ou d'actifs d'antiperspirant de chlorohydroxyde de zirconium, tels que le chlorhydrate d'aluminium, dans laquelle le sel de calcium soluble dans l'eau est le chlorure de calcium et dans laquelle le chlorure dans le chlorure de calcium peut être substitué en totalité ou en partie par du brome, de l'iode ou du nitrate.

2. Produit d'antiperspirant selon la revendication 1, dans lequel le sel d'alun est le sulfate de potassium aluminium.

3. Composition d'antiperspirant selon l'une quelconque des revendications précédentes présentant un rapport molaire de chlorure de calcium au sel d'alun qui est supérieur à 1:1.

4. Produit d'antiperspirant selon la revendication 3, dans lequel le rapport molaire de chlorure de calcium au sel d'alun est d'au moins 2:1.

5. Composition d'antiperspirant selon l'une quelconque des revendications précédentes, dans laquelle le sel d'alun présente une teneur en eau inférieure à 35 % en poids, de préférence inférieure à 28 % en poids et encore mieux inférieure à 20 % en poids.

6. Composition d'antiperspirant selon l'une quelconque des revendications précédentes, dans laquelle le sel de calcium soluble dans l'eau présente une teneur en eau inférieure à 15 % et de préférence inférieure à 8 %.

7. Composition d'antiperspirant selon l'une quelconque des revendications précédentes, dans laquelle un matériau de support liquide comprend 85 % en poids ou plus d'huiles présentant un paramètre de solubilité Hildebrand de 18,02 (MPa)^{0,5} ou inférieur.

8. Composition d'antiperspirant selon la revendication 7, dans laquelle un matériau de support liquide comprend 90 % en poids ou plus d'huiles présentant un paramètre de solubilité Hildebrand de 18,02 (MPa)^{0.5} ou inférieur.

9. Composition d'antiperspirant selon l'une quelconque des revendications précédentes, laquelle est une composition d'aérosol comprenant un propulseur.

10. Composition d'antiperspirant selon l'une quelconque des revendications précédentes, laquelle est une composition solide molle ou en stick comprenant un structurant.

11. Procédé non-thérapeutique de réduction de la transpiration du corps humain comprenant l'application topique d'une composition selon l'une quelconque des revendications précédentes.

12. Procédé de fabrication d'une composition d'antiperspirant anhydre présentant moins de 2 % en poids d'eau libre comprenant la réduction de la teneur en eau d'un sel d'alun (par lequel on indique du sulfate d'aluminium ou tout sulfate double d'aluminium et d'un métal univalent choisi parmi le potassium, le sodium, ou l'ammonium) et la combinaison du sel d'alun déshydraté résultant avec un sel de calcium soluble dans l'eau et un matériau de support liquide comprenant 80 % en poids ou plus d'huiles présentant un paramètre de solubilité Hildebrand de 18,02 (MPa)^{0,5} ou inférieur et l'exclusion d'aluminium synthétique et/ou d'actifs d'antiperspirant de chlorohydroxyde d'aluminium, tels que le chlorhydrate d'aluminium, dans lequel le sel de calcium soluble dans l'eau est le chlorure de calcium et dans lequel le chlorure dans le chlorure de calcium peut être substitué en totalité ou en partie par du brome, de l'iode ou du nitrate.

13. Procédé selon la revendication 12, dans lequel le sel d'alun est réduit en teneur en eau à moins de 28 % en poids avant sa combinaison avec le sel de calcium soluble dans l'eau et un matériau de support liquide.
